# EUROPÄISCHE PATENTSCHRIFT

(11) **EP 1 057 805 B1**
(45) Veröffentlichungstag und Bekanntmachung des Hinweises auf die Patenterteilung: **02.01.2004**
(21) Anmeldenummer: 00109344.2
(22) Anmeldetag: 02.05.2000
(51) Int. Cl.: C07C 67/04, C07C 69/013

(54) **Verfahren zur Herstellung von Isobornylmethacrylat**
Process for the preparation of isobornyl(meth)acrylate
Procédé de préparation du (méth)acrylate d'isobornyle

(30) Priorität: 06.05.1999 DE 19920796
(43) Veröffentlichungstag der Anmeldung: 06.12.2000
(73) Patentinhaber: Röhm GmbH & Co. KG, 64293 Darmstadt (DE)
(72) Erfinder: Knebel, Joachim, Dr., 64665 Alsbach-Hähnlein (DE); Saal, Doris, 64625 Bensheim (DE)

(56) Entgegenhaltungen:
- US-A- 3 087 962
- US-A- 5 719 314
- DATABASE WPI Section Ch, Week 197945 Derwent Publications Ltd., London, GB; Class A82, AN 1979-81642B XP002149924 & JP 54 126293 A (OSAKA YUKI KAGAKU KOGYO KK), 1. Oktober 1979 (1979-10-01) & CHEMICAL ABSTRACTS, vol. 92, no. 8, 25. Februar 1980 (1980-02-25) Columbus, Ohio, US; abstract no. 60517,

## Beschreibung

Die vorliegende Erfindung betrifft Verfahren zur Herstellung von Isobornyl(meth)acrylat durch die Umsetzung von Camphen mit (Meth)acrylsäure in Gegenwart von Schwefelsäure und wenigstens einer inhibierend wirkenden Verbindung.

Isobornyl(meth)acrylat findet als Monomer breite Anwendung bei der Herstellung von Lackbindemitteln. Hergestellt wird diese Verbindung beispielsweise durch die Umsetzung von Camphen mit (Meth)acrylsäure in Gegenwart eines sauren Kationenaustauscherharzes. Diese Art der Herstellung wird z.B. in EP-A-0 718 271 (Atochem) und DE-OS-44 19 686 (Hoechst AG) beschrieben. Die auf den ersten Blick elegante Ionenaustauscherkatalyse hat in der industriellen Praxis den Nachteil, daß der Katalysator beim wiederholten Einsatz an Aktivität verliert. Dies kann beispielsweise darauf zurückgeführt werden, daß selbst bei guter Prozeßführung auf dem Harz ein Polymerfilm gebildet wird. Auch eine erneute Reinigung und Aktivierung z.B. durch Waschen mit Säure und anschließendes Trocknen mit einem geeigneten Lösungsmittel, wie beispielsweise Aceton, führt nicht zum ursprünglichen Aktivitätsniveau zurück, so daß der Katalysator nach wenigen Einsatzzyklen ersetzt werden muß.

Des weiteren ist bekannt, Camphen mit (Meth)acrylsäure in Gegenwart von konzentrierter Schwefelsäure zu Isobornyl(meth)acrylat umzusetzen. Die Verwendung von konzentrierter Schwefelsäure als Katalysator wird beispielsweise in JP-OS-54/126293 beschrieben. Hierzu ist zunächst zu bemerken, daß die beschriebene Ausbeute von ca. 80 % bei weitem nicht erreicht werden konnte (siehe Vergleichsversuch 2).

Darüber hinaus muß, um eine unerwünschte Polymerisation des (Meth)acrylats zu verhindern, eine sehr große Menge an N, N'- Diphenyl-p-phenylendiamin eingesetzt werden (ca. 8%, bezogen auf die Acrylsäure). Dieser Inhibitor muß aber zum aller größten Teil aus der Mischung entfernt werden, da der Inhibitor eine gewünschte Polymerisation des Isobornyl(meth)acrylats verhindert.

Des weiteren besitzt der vorgeschlagene Inhibitor eine gelbe Farbe, so daß schon aus diesem Grund eine möglichst vollständige Abtrennung notwendig ist. Die notwendigen Aufreinigungsschritte, d.h. beispielsweise mehrfaches Destillieren, erniedrigt die Ausbeute auf sehr geringe Mengen an reinem Produkt. Versucht man, diesen Inhibitor durch andere Inhibitoren oder Mischungen von inhibierend wirkenden Verbindungen zu ersetzen, so erhält man jedoch nur einen Umsatz von ca. 10%.

In Anbetracht des hierin angegebenen und diskutierten Standes der Technik war es Aufgabe der vorliegenden Erfindung, ein Verfahren zur Herstellung von Isobornyl(meth)acrylat durch Umsetzung von Camphen mit (Meth)acrylsäure in Gegenwart von Schwefelsäure anzugeben, bei dem hohe Umsätze erzielt werden, ohne daß große Mengen an schwer abzutrennendem N,N'-Diphenyl-p-phenylendiamin eingesetzt werden müssen.

Eine weitere Aufgabe der Erfindung bestand darin, eine Inhibitorkombination zu finden, die in besonders geringen Mengen wirksam ist.

Des weiteren lag der Erfindung die Aufgabe zugrunde, ein Verfahren zur Destillation von Isobornyl(meth)acrylat aus einer Schwefelsäure enthaltenden Mischung anzugeben, ohne daß das Isobornyl(meth)acrylat polymerisiert.

Gelöst werden diese Aufgaben durch Verfahren zur Herstellung von Isobornyl(meth)acrylat mit allen Merkmalen der unabhängigen Verfahrensansprüche.

Vorteilhafte Ausgestaltungen des erfindungsgemäßen Verfahrens sind Gegenstand der auf die unabhängigen Verfahrensansprüche rückbezogenen Ansprüche.

Beim ersten Aspekt der vorliegenden Erfindung gelingt es dadurch, daß eine wäßrige Schwefelsäure mit einer Säurekonzentration im Bereich von 65 bis 85 Gew.-% eingesetzt wird, ein Verfahren zur Herstellung von Isobornyl(meth)acrylat durch Umsetzung von Camphen mit (Meth)acrylsäure in Gegenwart von Schwefelsäure und wenigstens einer inhibierend wirkenden Verbindung zur Verfügung zu stellen, durch welches das gewünschte Isobornyl(meth)acrylat in hoher Ausbeute und in reiner Form besonders kostengünstig erhalten wird.

Beim zweiten Aspekt der vorliegenden Erfindung gelingt es dadurch, daß man die Destillation in Gegenwart von 2,6-Di-tert.butyl-α-(dialkylamino)-p-cresol durchführt, ein Verfahren zur Destillation von Isobornyl(meth)acrylat aus einer Schwefelsäure enthaltenen Mischung zur Verfügung zu stellen, ohne daß ein großer Anteil des Isobornyl(meth)acrlyats im Destillationskolben polymerisiert.

Dabei ist besonders hervorzuheben, daß bereits durch jeden einzelnen der beiden Aspekte die der Erfindung zugrundeliegenden Aufgaben in hervorragender Weise gelöst werden.

Durch die erfindungsgemäßen Maßnahmen werden u.a. insbesondere folgende Vorteile erzielt:
⇒ Erfindungsgemäße Verfahren führen zu sehr hohen Umsätzen und einer hohen Reinheit der Produkte.
⇒ In dem erfindungsgemäßen Verfahren können kommerziell erhältliche Inhibitoren eingesetzt werden.
⇒ Das Verfahren kann kostengünstig durchgeführt werden, da keine teuren Ionenaustauscherharz eingesetzt werden müssen.
⇒ Des weiteren werden in besonders bevorzugten Ausführungsformen so geringe Mengen an inhibierend wirkenden Verbindungen benötigt, daß diese vollständig ohne größere Ausbeuteverluste entfernt werden können.

Die Schreibweise (Meth)acrylsäure umfaßt Methacrylsäure, Acrylsäure sowie Mischungen aus beiden Säuren.

Im Rahmen der vorliegenden Erfindung herstellbares Isobornyl(meth)acrylat läßt sich im allgemeinen durch die Formel (I) 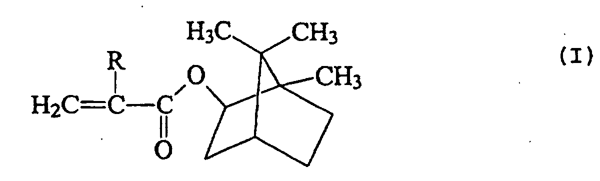 darstellen, worin der Rest R Wasserstoff oder eine Methylgruppe bedeutet.

Von Formel (I) werden insbesondere auch isomere Formen von Isobornyl(meth)acrylat umfaßt, die sich beispielsweise durch die Stellung des Säurerestes am Isobornylring ergeben können.

Die Verbindungen Camphen (2,2-Dimethyl-3-methylenbicyclo[2.2.1]heptan), Methacrylsäure und Acrylsäure sind in der Fachwelt weithin bekannt. Diese Verbindungen können von einer Vielzahl von Anbietern kommerziell erhalten werden.

Gleiches gilt für wäßrige Schwefelsäure, die in dem erfindungsgemäßen Verfahren in einer Säurekonzentration im Bereich von 65 bis 85 Gew.-% bevorzugt 70 bis 80 Gew.-% eingesetzt wird.

Um eine Polymerisation des Isobornyl(meth)acrylats bzw. der (Meth)acrylsäure zu verhindern, ist es notwendig, bei dem erfindungsgemäßen Verfahren wenigstens eine inhibierend wirkende Verbindung einzusetzen. Diese Verbindungen sind in der Fachwelt weithin bekannt. Erfindungsgemäß werden Hydrochinonalkylether, sterisch gehinderte Phenole und/oder sterisch gehinderte Piperidin-N-Oxylderivate eingesetzt.

Hydrochinonalkylether lassen sich mit der allgemeinen Formel (II) wiedergeben 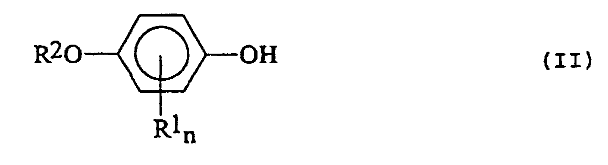 worin
R¹ Wasserstoff, einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, Cl, F oder Br; n eine ganze Zahl im Bereich von null bis vier, vorzugsweise null, eins oder zwei ist;
und
R² Wasserstoff, einen linearen oder verzweigten Alkylrest mit eins bis acht Kohlenstoffatomen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl oder tert.-Butyl.

Bevorzugte sterisch gehinderte Phenole sind im allgemeinen durch die allgemeine Struktur (III) darstellbar 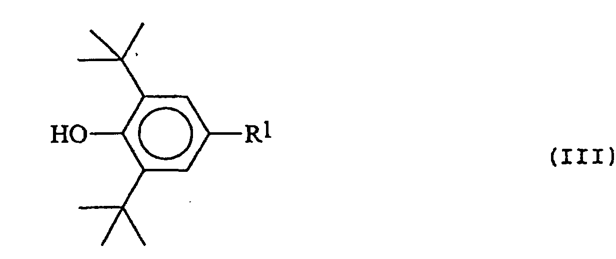 worin
R¹ einen linearen oder verzweigten Alkylrest mit 1 bis 8 Kohlenstoffatomen, Aryl oder Aralkyl, Proprionsäureester mit 1 bis 4 wertigen Alkoholen, welche auch Heteroatome wie S, O und N enthalten können, vorzugsweise einen Proprionsäureester mit Octadecylrest, bedeutet.

Eine weitere vorteilhafte Substanzklasse stellen gehinderte Phenole auf Basis von Triazinderivaten der Formel (IV) dar 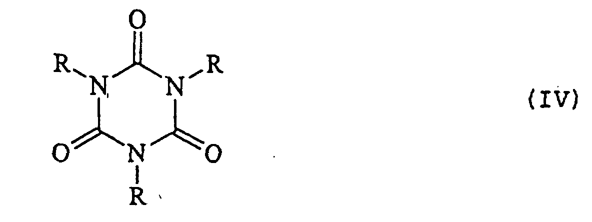 mit R = Verbindung der Formel (V) 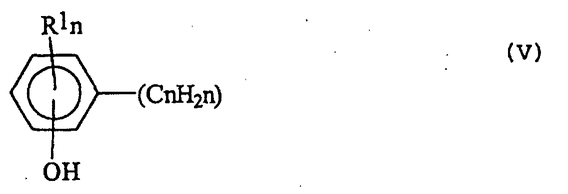 worin
R¹ = CₙH₂ₙ₊₁
mit n = 1 oder 2 ist.

Sterisch gehinderte Piperidin-N-Oxylderivate lassen sich beispielsweise durch die Formel (VI) darstellen 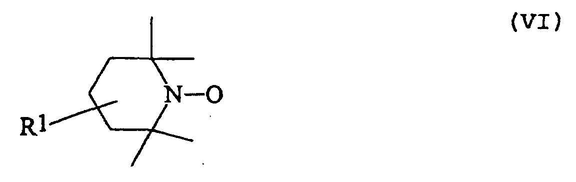 worin R¹ Hydroxy, einen Ether- oder einen Esterrest darstellt, wobei die letzteren Reste bis zu 20 Kohlenstoffatome aufweisen können. Über den Rest R¹ können auch mehrere sterisch gehinderte Piperidin-N-Oxyl-Reste verbunden werden.

Darüber hinaus können auch Verbindungen mit 1,4-Benzochinon als Stammverbindung eingesetzt werden. Diese lassen sich mit der Formel (VII) beschreiben 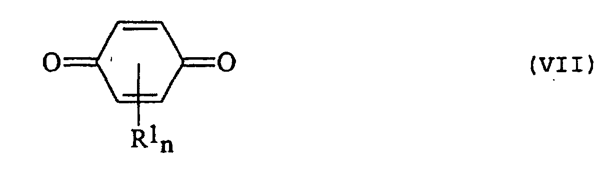 worin
R¹ einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, Cl, F oder Br; und
n eine ganze Zahl im Bereich von eins bis vier, vorzugsweise eins oder zwei ist.

Besonders erfolgreich werden die Verbindungen 1,4-Dihydroxybenzol, 4-Methoxyphenol, 2,5-Dichloro-3,6-dihydroxy-1,4-benzochinon, 1,3,5-Trimethyl-2,4,6-tris-(3,5-di-tert.butyl-4-hydroxybenzyl)benzol, 2,6-Di-tert.butyl-4-methylphenol, 2,4-Dimethyl-6-tert. butylphenol, 2,2-Bis [3,5-Bis (1,1-dimethylethyl)-4-hydroxyphenyl-1-oxoperopoxymethyl)1,3-propandiylester, 2,2'-Thiodiethylbis-[3-(3,5-di-tert.butyl-4-hydroxyphenyl)propionat, Octadecyl-3-(3,5-di-tert.butyl-4-hydroxyphenyl)propionat, 3,5-Bis(1,1-dimethylethyl-2,2-methylenbis-(4-methyl-6-tert.butyl)phenol, Tris-(4-tert.butyl-3-hydroxy-2,6-dimethylbenzyl)-s-triazin-2,4,6-(1H,3H,5H)trion, Tris (3,5-ditert.butyl-4-hydroxy)-s-triazin-2,46-(1H, 3H,5H) trion, tert.-Butyl-3,5-dihydroxybenzol, 2,6-Di-tert.butyl-α-(dimethylamino)-p-cresol, 4-Hydroxy-2,2,6,6-tetramethylpiperidin-N-oxyl und N,N'-Diphenyl-p-phenylendiamin eingesetzt. Ganz besonders erfolgreich können auch Mischungen dieser Substanzen verwendet werden.

Inhibitoren mit hohem Siedepunkt werden besonders bevorzugt eingesetzt, da diese durch Destillation besonders leicht abgetrennt werden können. Die Abtrennung der während der Umsetzung gegenwärtigen inhibierend wirkenden Verbindungen von dem Endprodukt ist sehr wichtig, da ansonsten die Initiatormenge zur Polymerisation des Isobornyl(meth)acrylats variieren könnte. Bei einer zu hohen Menge an Inhibitor findet selbst bei sehr großen Initiatorkonzentrationen keine Polymerisation mehr statt. Neben wirtschaftlichen Überlegungen ist dies ein wichtiger Grund, die Menge an inhibierend wirkenden Verbindungen möglichst niedrig zu wählen. Die Mindestmenge an inhibierend wirkenden Substanzen ergibt sich aus deren Fähigkeit als Radikalfänger zu wirken. Anhaltspunkte hierzu können beispielsweise von den Herstellern erhalten werden, wobei der Fachmann durch eine sehr begrenzte Zahl von Routineexperimenten geeignete Mengen ermitteln kann.

Ganz besonders überraschend wurde durch umfangreiche Versuche festgestellt, daß bei der kombinierten Verwendung von mindestens einem Hydrochinonalkylether, einem sterisch gehinderten Phenol und einem sterisch gehinderten Piperidin-N-oxyl-Derivat die Mengen der einzelnen Verbindungen besonders niedrig gewählt werden können.

So genügen beispielsweise bereits 100 bis 500 ppm Hydrochinonmonomethylether, 500 bis 2000 ppm Octadecyl-3-(3,5-di-tert.butyl-4-hydroxyphenyl) propionat und 5 bis 30 ppm, jeweils bezogen auf die Gesamtmenge der Reaktionsmischung, 4-Hydroxy-2,2,6,6-tetramethylpiperidin-N-oxyl, um eine unerwünschte Polymerisation während der Umsetzung zu verhindern.

Eisenionen können auf die erfindungsgemäße Umsetzung einen nachteiligen Effekt haben. Um sicherzustellen, daß die Konzentration an Eisenionen möglichst gering ist, kann ein Chelatbildner eingesetzt werden. Dies ist insbesondere dann vorteilhaft, wenn technische Schwefelsäure oder ein Edelstahlreaktor eingesetzt wird. Chelatbildner sind in der Fachwelt weithin bekannt. Als besonders bevorzugt hat sich die Verwendung von Nitrilotriessigsäure, Ethylendiamintetraessigsäure, N-(2-Hydroxyethyl)ethylendiamintri- , essigsäure, 1,2-Cyclo-hexylendinitrilotetraessigsäure, Diethylentriaminpentaessigsäure, 3,6-Di-oxaoctamethylendinitrilotetraessigsäure und/oder ein Alkalimetallsalz, wie beispielsweise ein Lithium-, Natrium-, Kalium- und/oder Rubidiumsalz, dieser Säuren erwiesen. Diese Substanzen können relativ kostengünstig erhalten werden.

Die Menge, in denen der Chelatbildner eingesetzt wird, ist von der möglichen Eisenionenkonzentration abhängig. Im allgemeinen ist jedoch eine Menge größer 500 ppm, bevorzugt größer 1000 ppm, bezogen auf die Gesamtmenge der Reaktionsmischung, ausreichend. Die erf indungsgemäße Reaktion kann unter Normal-, Untersowie Überdruck stattfinden. Dementsprechend können die Reaktionstemperaturen in einem weiten Bereich gewählt werden, die im allgemeinen vom eingesetzten Druck abhängig sind. Vorteilhafte Temperaturen liegen beispielsweise im Bereich von 50 bis 90 °C, insbesondere von 60 bis 80 °C.

Die Reaktion kann sowohl kontinuierlich als auch chargenweise durchgeführt werden. Das erfindungsgemäße Verfahren kann in Substanz, d.h. ohne Verwendung eines weiteren Lösungsmittels durchgeführt werden. Falls gewünscht, kann auch ein inertes Lösungsmittel eingesetzt werden. Hierzu gehören u.a. Benzol, Toluol, n-Hexan, Cyclohexan, Methylisobutylketon sowie Methylethylketon.

Die Reaktionszeiten sind beispielsweise von den gewählten Parametern wie Druck und Temperatur abhängig. Sie liegen aber in vielen Fällen im Bereich von ein bis 12 Stunden, bevorzugt von drei bis acht Stunden.

Die Aufreinigung der Reaktionsmischung kann auf jede dem Fachmann bekannten Weise erfolgen. Besonders bevorzugt ist jedoch eine Destillation gemäß dem zweiten Aspekt der vorliegenden Erfindung. Hierbei wird die Destillation in Gegenwart von 2,6-Di-tert.buyl-α-(dialkylamino)-p-cresol durchgeführt. Bevorzugt ist hierbei 2,6-Di-tert.butyl-α-(dimethylamino)-p-cresol. Diese inhibierend wirkende Substanz wird vorzugsweise in einer Menge im Bereich von 250 bis 1000 ppm, bezogen auf die Gesamtmenge der Reaktionsmischung, eingesetzt. Es ist zwar durchaus möglich größere Mengen einzusetzen, falls Sorge dafür getragen wird, daß diese Menge nicht in die Destillationsvorlage überführt wird. Dies ist jedoch wirtschaftlich nicht sinnvoll.

Zusätzlich zu dem p-Cresol können weitere inhibierend wirkende Verbindungen beigefügt werden. Hierzu gehören insbesondere die weiter oben genannten Inhibitoren. Die Destillation wird vorzugsweise bei Unterdruck, d.h. einem Druck kleiner 200 mbar, bevorzugt kleiner 150 mbar und ganz bevorzugt kleiner 130 mbar durchgeführt, um die Temperaturbelastung des Endprodukts möglichst gering zu halten. Die Destillationstemperatur ist von dem eingesetzten Druck abhängig. Je niedriger der Druck desto geringer die benötigte Temperatur. Bevorzugt ist die Destillationstemperatur kleiner 150 °C.

Das folgende Beispiel und das Vergleichsbeispiel dient zur detaillierten Beschreibung der vorliegenden Erfindung, ohne daß hierdurch eine Einschränkung erfolgen soll. Die Angaben in % beziehen sich auf das Gesamtgewicht, es sein denn anderes ist vermerkt.

### Beispiel 1

In einem 500 ml Vierhalskolben mit mechanischem Rührer, Innenthermometer, aufgesetztem Rückflußkühler und Gaseinleitrohr zur Lufteinleitung legt man 238 g (1,75 mol) Camphen vor und gibt dann 166 g (1,925 mol) (Meth)acrylsäure sowie als inhibierend wirkende Verbindung 0,08 g (200 ppm) Hydrochinonmonomethylether, 0,4 g (1000 ppm) Octadecyl-3-(3,5-di-tert.butyl-4-hydroxyphenyl)propionat, 0,008 g (20 ppm) 4-Hydroxy-2,2,6,6-tetramethylpipiderin-N-oxyl sowie 0,4 g ( 1000 ppm) Titriplex III (Ethylendinitrilotetraessigsäuredinatriumsalz, Merck) hinzu. Unter Rühren und Lufteinleitung versetzt man mit 10,7 g (0,082 mol) 75%iger Schwefelsäure, wobei sich der Ansatz braun verfärbt. Dann wird auf 70 °C erhitzt und 4,5 Stunden gerührt, wobei sich ein Endumsatz von 70 % ergibt (ermittelt durch die Titration mit ethanolischer KOH gegen Phenolphthalein).

Der Ansatz wird auf Raumtemperatur abgekühlt und mit 5 g (0,09 mol) Calciumoxid unter Rühren innerhalb von 30 Minuten neutralisiert. Dann gibt man weitere 8 mg (20 ppm ) 4-Hydroxy-2,2,6,6-tetramethylpiperidin-N-oxyl, 0,2 g (500 ppm) N,N'-Diphenyl-p-phenylendiamin und 0,2 g (500 ppm) 2,6-Di-tert.butyl-α-(dimethylamino)-p-cresol hinzu und reinigt den unfiltrierten Rohester durch Destillation über eine 20 cm Vigreux-Kolonne bei einer Temperatur innerhalb des Destillationskolbens von 100 bis 140 °C und einem Druck von 130 bis 5 mbar, wobei der Druck abnehmend der Destillationsrate angepaßt wird, so daß eine gleichbleibende Destillationsrate erhalten wird. Man erhält nun 35 g Vorlauf, der gemäß GC 62,8 % Camphen, 26,9 % (Meth)acrylsäure und 7,6 % Isobornyl(meth)acrylat aufweist, sowie 234 g (Umsatzausbeute 93,6 %) Isobornyl(meth)acrylat mit einer gaschromatographisch ermittelten Reinheit von 97,6 %.

### Vergleichsbeispiel 1

Das Vergleichsbeispiel 1 wurde wie das Beispiel 1 durchgeführt, außer daß 8,08 g (0,083 mol) konzentrierter Schwefelsäure anstatt der 75%igen Schwefelsäure eingesetzt wurden. Man erhält bei der destillativen Aufreinigung des Rohesters 51 g Vorlauf, der gemäß GC aus 71,4 % Camphen, 13,7 % (Meth)acrylsäure und weiteren unbekannten Produkten besteht, und 24 g Hauptlauf, der gemäß GC 14,6 % Camphen, 67 % (Meth)acrylsäure und 13,3 % Isobornyl(meth)acrylat enthält, wobei die Destillation zum Erliegen kommt. Die Mischung im Destillationskolben wird hierbei sehr viskos, was auf eine Polymerisation hinweist.

### Vergleichsbeispiel 2

Dieses Vergleichsbeispiel wurde entsprechend dem Synthesebeispiel 1 der JP-OS 54/1266293 durchgeführt. ' Zu 272 g Camphen werden 176 g (2,08 mol) Methacrylsäure und 12 g (27000 ppm) N,N'-Diphenyl-p-phenylendiamin gegeben. Anschließend werden hierzu insgesamt 6,5 g (0,065 mol) konzentrierte Schwefelsäure (98%) langsam zugetropft und weitere 2 Stunden bei Raumtemperatur gerührt. Danach wurde die Reaktionsmischung mit 6,9 g (0,065 mol) Natriumcarbonat neutralisiert.

Die Analyse der Mischung mit GC-Chromatographie: 69,37 % Camphen, 22,29 % Methacrylsäure, 1,49 % iso-Borneol und 6,39 % Isobornylmethacrylat. Da nahezu kein Umsatz stattgefunden hat, wurde nicht weiter aufgearbeitet. Die behauptete Ausbeute ist mit der beschriebenen Methode nicht erzielbar, wobei insbesondere darauf verwiesen werden sollte, daß ein Großteil der Schwefelsäure durch N,N'-Diphenyl-p-phenylendiamin neutralisiert wird (12 g entsprechen 0,046 mol, so daß nur 0,019 mol Schwefelsäure als Katalysator zur Verfügung stehen).

## Patentansprüche

1. Verfahren zur Herstellung von Isobornyl(meth)acrylat durch die Umsetzung von Camphen mit (Meth)acrylsäure in Gegenwart von Schwefelsäure und wenigstens einer inhibierend. wirkenden Verbindung,
**dadurch gekennzeichnet, daß**
eine wäßrige Schwefelsäure mit einer Säurekonzentration im Bereich von 65 bis 85 Gew.-% und einer Menge von etwa 0,0426 mol pro Mol Methacrylsäure eingesetzt wird und daß man als inhibierend wirkende Verbindung einen Hydrochinonalkylether, ein sterisch gehindertes Phenol und/oder ein sterisch gehindertes Piperidin-N-oxyl-Derivat einsetzt.

2. Verfahren gemäß Anspruch 1,
**dadurch gekennzeichnet, daß**
eine wäßrige Schwefelsäure mit einer Säurekonzentration im Bereich von 70 bis 80 Gew.-% eingesetzt wird.

3. Verfahren gemäß einem oder mehreren der vorhergehenden Ansprüche,
**dadurch gekennzeichnet, daß**
man als inhibierend wirkende Verbindung Hydrochinonmonomethylether, Octadecyl-3-(3,5-di-tert.butyl-4-hydroxyphenyl)propionat oder 4-Hydroxy-2,2,6,6-tetramethylpiperidin-N-oxyl sowie Mischungen dieser Verbindungen einsetzt.

4. Verfahren gemäß einem oder mehreren der vorhergehenden Ansprüche,
**dadurch gekennzeichnet, daß**
man zusätzlich einen Chelatbildner einsetzt.

5. Verfahren gemäß Anspruch 5,
**dadurch gekennzeichnet, daß**
man als Chelatbildner Nitrilotriessigsäure, Ethylendiamintetraessigsäure, N-(2-Hydroxyethyl)-ethylendiamintriessigsäure, 1,2-Cyclohexylendinitrilotetraessigsäure, Diethylentriaminpentaessigsäure, 3,6-Dioxaoctamethylendinitrilotetraessigsäure und/oder ein Alkalimetallsalz dieser Säuren einsetzt.

6. Verfahren gemäß einem oder mehreren der vorhergehenden Ansprüche,
**dadurch gekennzeichnet, daß**
die Umsetzung bei einer Temperatur im Bereich von 50 bis 90 °C stattfindet.

7. Verfahren gemäß einem oder mehreren der vorhergehenden Ansprüche,
**dadurch gekennzeichnet, daß**
das Isobornyl(meth)acrylat nach der Umsetzung durch Destillation in Gegenwart von 2,6-Di-tert.butyl-α-(dimethylamino)-p-cresol isoliert wird.

8. Verfahren zur Destillation von Isobornyl(meth)acrylat aus einer Schwefelsäure enthaltenden Mischung,
**dadurch gekennzeichnet, daß**
die Destillation in Gegenwart von 2,6-Di-tert.butyl-α-(dialkylamino)-p-cresol durchgeführt wird.

9. Verfahren gemäß Anspruch 9,
**dadurch gekennzeichnet, daß** man als 2,6-Di-tert.butyl-α-(dialkylamino)-p-cresol 2,6-Di-tert.butyl-α-(dimethylamino)-p-cresol einsetzt.

## Claims

1. Process for preparing isobornyl(meth)acrylate by reacting camphene with (meth)acrylic acid in the presence of sulphuric acid and at least one compound with an inhibiting effect, **characterised in that** an aqueous sulphuric acid with an acid concentration in the range from 65 to 85 wt.% and a quantity of about 0.0426 mol per mol of methacrylic acid is used and that a hydroquinone alkylether, a sterically hindered phenol and/or a sterically hindered piperidine-N-oxyl derivative is used as the compound with an inhibiting effect.

2. Process according to claim 1, **characterised in that** an aqueous sulphuric acid with an acid concentration in the range from 70 to 80 wt.% is used.

3. Process according to one or more of the preceding claims, **characterised in that** hydroquinone monomethylether, octadecyl-3-(3,5-di-tert.butyl-4-hydroxyphenyl)propionate or 4-hydroxy-2,2,6,6-tetramethylpiperidine-N-oxyl and mixtures of these compounds are used as the compound with an inhibiting effect.

4. Process according to one or more of the preceding claims, **characterised in that** a chelating agent is additional used.

5. Process according to claim 5, **characterised in that** nitrilotriacetic acid, ethylenediaminetetraacetic acid, N-(2-hydroxyethyl)-ethylenediaminetriacetic acid, 1,2-cyclohexylendinitrilotetraacetic acid, diethylenetriaminepentaacetic acid, 3,6-dioxaoctamethylenedinitrilotetraacetic acid and/or an alkali metal salt of these acids is used as chelating agent.

6. Process according to one or more of the preceding claims, **characterised in that** the reaction takes place at a temperature in the range from 50 to 90°C.

7. Process according to one or more of the preceding claims, **characterised in that** the isobornyl(meth)acrylate is isolated after the reaction by distillation in the presence of 2,6,-di-tert.butyl-α-(dimethylamino)-p-cresol.

8. Process for distilling isobornyl(meth)acrylate from a mixture containing sulphuric acid, **characterised in that** the distillation is carried out in the presence of 2,6-di-tert.butyl-α-(dialkylamino)-p-cresol.

9. Process according to claim 9, **characterised in that** 2,6-di-tert.butyl-α-(dialkylamino)-p-cresol is used as the 2,6-di-tert.butyl-α-(dialkylamino)-p-cresol.

## Revendications

1. Procédé de production de (méth)acrylate d'isobornyle par la réaction du camphène avec l'acide (méth)acrylique en présence d'acide sulfurique et d'au moins un composé à action inhibitrice,
**caractérisé en ce qu'**
on met en oeuvre un acide sulfurique aqueux ayant une concentration en acide dans la plage de 65 à 85 % en poids et une quantité d'environ 0,0426 mol par mol d'acide méthacrylique, et on met en oeuvre comme composé à action inhibitrice un éther d'alkyle d'hydroquinone, un phénol stériquement encombré et/ou un dérivé de piperidine N-oxyl stériquement encombré.

2. Procédé selon la revendication 1,
**caractérisé en ce qu'**
on met en oeuvre un acide sulfurique aqueux ayant une concentration en acide dans la plage 70 à 80 % en poids.

3. Procédé selon l'une ou plusieurs des revendications précédentes,
**caractérisé en ce qu'**
on met en oeuvre comme composé à action inhibitrice, de l'éther mono-méthylique d'hydroquinone, le 3-(3,5-ditert.butyl 4-hydroxyphényl) propionate d'octadécyle, ou le 4-hydroxy-2,2,6,6-tétraméthylpipéridine N-oxyle ainsi que des mélanges de ces composés.

4. Procédé selon l'une ou plusieurs des revendications précédentes,
**caractérisé en ce qu'**
on met en oeuvre en supplément un agent chélatant.

5. Procédé selon la revendication 4,
**caractérisé en ce qu'**
on met en oeuvre comme agent chélatant, de l'acide nitrilotriacétique, de l'acide éthylène diaminotétraacétique, l'acide N-(2-hydroxy l'éthyl)éthylène diaminotriacétique, l'acide 1,2-cyclohexylène dinitrilotétraacétique, l'acide diéthylènetriaminopentaacétique, l'acide 3,6-dioxaoctaméthylènedinitrilotétraacétique et/ou un sel de métal alcalin de ces acides.

6. Procédé selon l'une ou plusieurs des revendications précédentes,
**caractérisé en ce que**
la réaction se produit à une température dans la plage de 50 à 90°C.

7. Procédé selon l'une ou plusieurs des revendications précédentes,
**caractérisé en ce qu'**
on isole le (méth)acrylate d'isobornyle après la réaction, par distillation en présence de 2,6-ditert.butyl α-(diméthylamino)-p-crésol.

8. Procédé de distillation de (méth)acrylate d'isobornyle à partir d'un mélange contenant de l'acide sulfurique,
**caractérisé en ce que**
la distillation est effectuée en présence de 2,6-ditert.butyl α-(dialkylamino)-p-crésol.

9. Procédé selon la revendication 8,
**caractérisé en ce qu'**
on met en oeuvre comme 2,6-diterbutyl α-(dialkylamino)-p-crésol, le 2,6-ditert.butyl α-(diméthylamino)-p-crésol.
